## Europäisches Patentamt

## European Patent Office

## Office européen des brevets

(19)

(11) Veröffentlichungsnummer: **0 056 159**
**B1**

(12) **EUROPÄISCHE PATENTSCHRIFT**

(45) Veröffentlichungstag der Patentschrift:
**19.02.86**

(21) Anmeldenummer: **81110813.3**

(22) Anmeldetag: **29.12.81**

(51) Int. Cl.⁴: **C 08 G 18/02**, C 07 D 251/34,
C 08 G 18/22, C 08 G 18/79,
C 08 G 18/80

(54) **Verfahren zur Herstellung von Isocyanuratgruppen aufweisenden Polyisocyanaten und ihre Verwendung bei der Herstellung von Polyurethanen.**

(30) Priorität: **08.01.81 DE 3100263**

(43) Veröffentlichungstag der Anmeldung:
**21.07.82 Patentblatt 82/29**

(45) Bekanntmachung des Hinweises auf die Patenterteilung:
**19.02.86 Patentblatt 86/8**

(84) Benannte Vertragsstaaten:
**BE DE FR GB IT**

(56) Entgegenhaltungen:
**DD - A - 105 813**
**DE - A - 3 006 634**
**DE - B - 1 954 093**
**GB - A - 1 433 642**
**US - A - 2 952 665**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Stemmler, Ingo, Dr., Buschweg 21, D-5068 Odenthal (DE)**
Erfinder: **Müller, Hanns Peter, Dr., Im Kerberich 6, D-5068 Odenthal (DE)**
Erfinder: **Wagner, Kuno, Dr., Am Kiesberg 8, D-5090 Leverkusen 1 (DE)**

ACTORUM AG

**Beschreibung**

Die Erfindung betrifft ein Verfahren zur Herstellung von Isocyanuratgruppen aufweisenden Polyisocyanaten durch Trimerisierung eines Teils der Isocyanatgruppen von organischen Polyisocyanaten unter Verwendung von neuartigen, nachstehend näher beschriebenen Trimerisierungskatalysatoren und Abbruch der Trimerisierungsreaktion durch Zugabe eines Katalysatorengifts, sowie die Verwendung der erfindungsgemässen Verfahrensprodukte, gegebenenfalls in von monomerem Ausgangspolyisocyanat befreiter und/oder in mit Blockierungsmitteln für Isocyanatgruppen blockierter Form als Isocyanatkomponente bei der Herstellung von Polyurethankunststoffen.

Verfahren zur Trimerisierung organischer Isocyanate, insbesondere Polyisocyanate sind in grosser Zahl bekannt (J.H. Saunders und K.C. Frisch, Polyurethanes Chemistry and Technology, S. 94 ff (1962)). Als Katalysatoren für die Trimerisierung sind starke organische Basen geeignet, so z.B. die alkalisch wirkenden Metallsalze von Carbonsäuren, Metallalkoholate, Metallphenolate, Alkalimetallcarbonate, tert. Amine, tert. Phosphine und die «Onium»-Verbindungen von Stickstoff und Phosphor sowie basische Heterocyclen dieser Elemente. Die Katalysatoren werden dabei häufig in Kombination eingesetzt oder zusammen mit weiteren Co-Katalysatoren wie Mono-N-substituierten Carbaminsäureestern (A. Farkas und G.A. Mills, Advances in Catalysis, Vol. 13, 393 (1962)). Man arbeitet meist mit aufwendigen Katalysatorsystemen da bekannt ist, dass einfache Metallsalze wie Carboxylate oder Alkoholate nur in relativ hoher Konzentration und bei hoher Temperatur eine Cyclotrimerisierung von Isocyanaten bewirken (vgl. z.B. GB-PS 809 809, Beispiel 6).

Will man die Trimerisierung mit Metallsalzen im Lösungsmittel durchführen, müssen stark polare aprotische Lösungsmittel verwendet werden wie Dimethylformamid oder Dimethylsulfoxid, da nur diese anorganische Metallsalze und Metallsalze mit kleinem organischem Rest zu lösen vermögen (DE-OS 2 839 084). Auch hier sind immer noch Katalysatorkonzentrationen von 0,1 bis 0,5 Gew.-% notwendig. Dies gilt auch, wenn man protische Lösungsmittel verwendet, die aber ihrerseits mit dem Isocyanat Urethane bilden und so die NCO-Zahl senken, bzw. Ausfällungen und Trübungen bilden, so dass das Reaktionsprodukt filtriert werden muss (GB-PS 920 080).

Ausserdem bewirken die Metallsalze des Standes der Technik nur bei aromatischen Isocyanaten eine schnelle Trimerisierung, aliphatische Mono- und Polyisocyanate benötigen eine hohe Katalysatorkonzentration sowie vergleichsweise hohe Temperaturen, wodurch häufig ein uneinheitlicher exothermer Reaktionsverlauf eintritt und bei Polyisocyanaten hochviskose, stark verfärbte Produkte entstehen (vgl. US-PS 3 330 828, Beispiel 1 bis 4; GB-PS 952 931, Beispiel 3; DE-AS 1 013 869, Beispiel 3), oder Gelteilchen gebildet werden (GB-PS 966 338, Beispiel 3), wodurch die Produkte als Isocyanatkomponente für hochwertige Polyurethanlacke wenig geeignet sind. Ein wesentlicher Nachteil der Metallsalz-Katalyse ist ausserdem in dem Umstand zu sehen, dass beim Abstoppen des Katalysators z.B. durch Zusatz saurer Verbindungen anorganische Salze entstehen, die im Polyisocyanat unlöslich sind und Trübungen verursachen. Bei den neueren Verfahren des Standes der Technik werden deshalb spezielle organische Basen als Trimerisierungskatalysatoren eingesetzt. So nimmt man z.B. zur Trimerisierung von aromatischen Polyisocyanaten Mannich-Basen (DE-OS 2 551 634 und DE-OS 2 641 380) oder tert. Phosphine, wobei im Falle der Phosphine zuerst Uretdione gebildet werden, die sich erst in zweiter Reaktionsphase zum Isocyanurat umsetzen (DE-OS 1 201 992). Zur Trimerisierung (cyclo)-aliphatischer Diisocyanate verwendet man neuerdings häufig organische Basen mit Betain-Struktur wie quartäre Ammoniumhydroxide (EP-A 010 589 und EP-A 009 694), Aminimide (J.E. Kresta, R.J. Chang, S. Kathiriya und K.C. Frisch, Makromol. Chem. 180, 1081 (1979)) sowie Aziridinderivate in Kombination mit tert. Aminen (DE-AS 2 325 826).

Bei allen diesen Katalysatorsystemen ist nachteilig, dass ganz bestimmte Temperaturintervalle eingehalten werden müssen, teils nur lösungsmittelfrei, teils nur in ausgewählten Lösungsmitteln gearbeitet werden kann und insbesondere entweder nur aromatische oder nur aliphatische Polyisocyanate trimerisiert werden können.

Aufgabe der vorliegenden Erfindung ist es daher, ein Verfahren zur Verfügung zu stellen, nach welchem in technisch einfacher Weise farblose Isocyanuratgruppen aufweisende Polyisocyanate sowohl aromatischer als auch aliphatischer Struktur im Lösungsmittel oder lösungsmittelfrei und ohne aufwendige Temperaturregelung mit ein und demselben Katalysator hergestellt werden können.

Diese Aufgabe konnte überraschenderweise mit dem nachstehend näher beschriebenen erfindungsgemässen Verfahren, bei welchem neuartige Trimerisierungskatalysatoren eingesetzt werden, gelöst werden.

Gegenstand der Erfindung ist ein Verfahren zur Herstellung von Isocyanuratgruppen aufweisenden Polyisocyanaten durch Trimerisierung eines Teils der Isocyanatgruppen von organischen Polyisocyanaten oder Gemischen aus Di- und Monoisocyanaten in Gegenwart von basischen Alkalimetallverbindungen als Katalysatoren und gegebenenfalls in Gegenwart von Hilfslösungsmitteln und Abbruch der Trimerisierungsreaktion durch Zugabe eines Katalysatorengiftes, dadurch gekennzeichnet, dass man als Trimerisierungskatalysatoren Komplexe aus

i) basischen Alkalimetallverbindungen und

ii) acyclischen organischen Verbindungen, die
 a) insgesamt mindestens 7 Ethylenoxid- und gegebenenfalls Propylenoxideinheiten in Form einer oder mehrerer Polyetherketten mit jeweils mindestens 3 Alkylenoxideinheiten, wobei mindestens 80% aller vorliegender Alkylenoxideinheiten Ethylenoxideinheiten darstellen,

b) einen Gehalt an innerhalb mindestens einer Polyetherkette angeordneten Alkylenoxideinheiten der genannten Art von insgesamt mindestens 40 Gew.-% und

c) ein Molekulargewicht von 326–1000 aufweisen,

in Mengen von 0,001 bis 0,1 Mol.-%, bezogen auf Alkalimetallverbindung (i) einerseits und zu trimerisierendes Ausgangspolyisocyanat andererseits, verwendet, mit der Massgabe, dass im Falle der Verwendung von Komplexbildnern (ii) mit freien Hydroxylgruppen bzw. an einwertigen Alkoholen als Lösungsmittel deren Menge so begrenzt wird, dass im Reaktionsgemisch maximal 2 Mol.-%, bezogen auf die Isocyanatgruppen des Ausgangspolyisocyanats, an Hydroxylgruppen vorliegen.

Gegenstand der Erfindung ist auch die Verwendung der erfindungsgemässen Verfahrensprodukte, gegebenenfalls in von überschüssigen Ausgangspolyisocyanaten befreiter Form und/oder gegebenenfalls in mit Blockierungsmitteln für Isocyanatgruppen blockierter Form als Isocyanatkomponente zur Herstellung von Polyurethanen nach dem Isocyanat-Polyadditionsverfahren.

Bei den neuartigen, beim erfindungsgemässen Verfahren als Trimerisierungskatalysatoren einzusetzenden Komplexen handelt es sich um solche auf Basis von (i) basischen Alkalimetallverbindungen und (ii) bestimmten, Polyethersegmente aufweisende organische Verbindungen.

Als Katalysatorkomponente (i) können beliebige basische Alkalimetallverbindungen eingesetzt werden, d.h. insbesondere solche, die in einmolarer Konzentration in wässriger Lösung einen pH-Wert der Lösung von mindestens 7,5 ergeben. Es handelt sich im allgemeinen um, dieser Definition entsprechende, Verbindungen des Natriums, Kaliums, Rhubidiums oder Cäsiums, wobei die Natrium- und Kaliumverbindungen bevorzugt sind. Lithiumverbindungen sind weniger bevorzugt.

Typische Beispiele von als Katalysatorkomponente (i) geeigneten basischen Alkalimetallverbindungen sind die Hydroxide, Carbonate, Cyanate, Cyanide, Carboxylate (mit 1 bis 18 Kohlenstoffatomen) wie z.B. Formiate, Acetate, Propionate, 2 Ethyl-hexanoate, Stearate, Oleate, Benzoate, Naphthenate, Caprolate, Alkoholate (mit 1–6 Kohlenstoffatomen) wie Methylate, Ethylate oder Butylate, Phenolate (mit 6–10 Kohlenstoffatomen), Enolate wie Acetylacetonate oder Acetessigesterate oder Anlagerungsprodukte an Säureamide wie Essigsäureamid der genannten Alkalimetalle. Die Carboxylate sind bevorzugt.

Bei der Katalysatorkomponente (ii) handelt es sich um acyclische organische Verbindungen, die die oben unter a) bis c) genannten Kriterien erfüllen.

Bevorzugte Katalysatorkomponenten (ii) sind solche, die

a) Polyetherketten mit jeweils mindestens 7 Alkylenoxideinheiten und

b) einen Gehalt an innerhalb von Polyetherketten eingebauten Alkylenoxideinheiten der genannten Art von mindestens 55 Gew.-% aufweisen, wobei mindestens 80% aller vorliegender Alkylenoxideinheiten Ethylenoxideinheiten darstellen.

Typische Beispiele für geeignete Katalysatorkomponenten (ii) sind diesen Definitionen entsprechende 1- bis 3-wertige Polyetheralkohole, wie sie in an sich bekannter Weise durch Alkoxylierung, insbesondere Ethoxylierung geeigneter Startermoleküle wie einwertigen Alkoholen wie Methanol, Ethanol, n- oder i-Propanol, n-, i-, sec.- oder tert.-Butanol von Wasser oder von mindestens zweiwertigen Startermolekülen wie Ethylenglykol, Propandiol-(1,2), Propandiol-(1,3), Butan-, Pentan- oder Hexandiolen, Glycerin, Trimethylolethan oder Trimethylolpropan erhalten werden können. Geeignet sind auch solche Polyether der beispielhaft genannten Art, deren endständige(n) Hydroxylgruppe(n) beispielsweise durch eine Alkylierung, Acylierung und/oder Urethanisierung verschlüsselt wurden, so dass keine Endgruppen mehr vorliegen, deren Reaktivität gegenüber Isocyanatgruppen mit der Reaktivität von Hydroxylgruppen vergleichbar ist, oder Derivate der genannten Polyetheralkohole, die durch Umsetzung mit Kettenverlängerungsmitteln, beispielsweise Diisocyanaten wie Tetramethylendiisocyanat, Hexamethylendiisocyanat oder 2,4-Diisocyanatotoluol kettenverlängert worden sind, soweit die so erhaltenen Derivate den oben gemachten Definitionen entsprechen.

Der Verschlüsselung der endständigen Hydroxylgruppe(n) der oben beispielhaft genannten Polyetheralkohole durch Alkylierung geschieht beispielsweise durch Umsetzung der Polyetheralkohole mit Alkylierungsmitteln wie z.B. Dimethylsulfat, $C_1$–$C_4$-Alkyl-Halogeniden oder Benzylhalogenid; die Verschlüsselung durch eine Acylierungsreaktion durch Umsetzung mit Acylierungsmitteln wie z.B. Essigsäureanhydrid, Acetylchlorid oder Benzoylchlorid; die Verschlüsselung durch Urethanisierung durch Umsetzung mit einwertigen Isocyanaten wie z.B. Methyl-, Ethyl-, Hexyl- oder Phenylisocyanat. Durch Umsetzung der beispielhaft genannten Polyetheralkohole mit Aldehyden wie Formaldehyd, Acetaldehyd oder Benzaldehyd im Sinne einer Acetalisierung werden gegebenenfalls substituierte Methylenoxideinheiten in die Polyether eingeführt.

Zur Herstellung der erfindungswesentlichen, als Trimerisierungskatalysatoren einzusetzenden Komplexe werden die beispielhaft genannten Komponenten (i) und (ii) bzw. beliebige ihrer Gemische in solchen Mengenverhältnissen miteinander umgesetzt, dass auf jedes Grammäquivalent an basischer Metallverbindung (i) 0,8 bis 2,0, vorzugsweise 1,0 bis 1,5 Mol an, obigen Definitionen entsprechenden Verbindungen (ii) zur Verfügung stehen. Falls bei der Herstellung der Komplexe Alkoxylierungsprodukte der beispielhaft genannten Startermoleküle bzw. deren Derivate eingesetzt werden, die wegen der statistisch verlaufenden Alkoxylierungsreaktion Gemische darstellen, die neben den obigen Definitionen entsprechenden Verbindungen (ii) auch diesen Definitionen, beispielsweise wegen eines zu geringen Gehalts an Alkylenoxideinheiten, nicht entsprechenden Verbindungen enthalten, muss die Menge solcher Gemische selbstverständlich so bemessen werden, dass obige Mengenverhältnisse der Komponenten (i) und (ii) eingehalten werden. Die Umsetzung zwischen den Komponenten (i) und (ii), d.h. die Komplexbildung, erfolgt im allgemeinen spontan bei einer Temperatur von ca. 10 bis 60 °C, insbesondere wenn miteinander verträgliche Komponenten (i) und (ii) in Abwesenheit von Lösungsmitteln eingesetzt werden. Die Bildung der Komplexe kann jedoch auch in Gegenwart von Lösungsmitteln bzw. Lösungsmittelgemischen erfolgen. Beispiele geeigneter Lösungsmittel sind Methanol, Ethanol, Propanol, Ethylenglykol, Propandiol-(1,2), Propandiol-(1,3), Butylenglykol, Glycerin oder Oligoethylen- bzw. -propylenglykole (Oligomerisierungsgrad 2 bis 6) (die Alkohole sollen einerseits die Metallverbindungen gut lösen, andererseits aber mit dem Isocyanat noch einigermassen mischbar sein und eine niedrige Viskosität besitzen).

Ausserdem können auch aprotische, gegenüber Isocyanaten unreaktive Lösungsmittel eingesetzt werden, deren $E_T$-Wert (Cr. Reichardt, Lösungsmittel-Effekte in der organischen Chemie, Chem. Taschenbücher Bd. 4, Verlag Chemie 196) vorzugsweise im Bereich 33,5 bis 47 liegt. Ausser Nitrilen, wie Acetonitril, Propionitril oder Benzonitril, Nitroverbindungen, wie Nitromethan oder Nitrobenzol, Kohlensäureestern, wie z.B. Ethylen- oder Propylencarbonat, oder Ketonen, wie Aceton, Acetophenon, Butyl-methyl-keton oder Isobutylmethyl-keton, können selbst so unpolare Lösungsmittel wie Chlorkohlenwasserstoffe, z.B. Methylenchlorid, Chloroform, 1,1,1-Trichlorethan oder Trichlorethylen oder aromatische Kohlenwasserstoffe, wie Benzol, Toluol oder Xylol oder Ester, wie Essigsäureethylester, Essigsäurebutylester oder Ethylenglykolmonomethyletheracetat eingesetzt werden.

Stark polare Lösungsmittel wie Dimethylformamid, N-Methylpyrrolidon, Tetramethylharnstoff oder Dimethylsulfoxid können zwar prinzipiell auch verwendet werden, ihr Einsatz ist jedoch nicht zu empfehlen, da sie einerseits nur schwierig von Nebenprodukten wie z.B. Aminen zu reinigen sind und andererseits im allgemeinen unerwünschte Nebenreaktionen von NCO-Gruppen katalysieren, so dass die erfindungsgemässen Verfahrensprodukte bei Anwesenheit derartiger Lösungsmittel nicht lagerstabil sein würden.

Hilfslösungsmittel der beispielhaft genannten Art werden im allgemeinen dann eingesetzt, wenn die Komponenten (i) und (ii) nicht oder nur schlecht miteinander verträglich sind. Falls die Komponenten (i) und (ii) miteinander verträglich und/oder in dem verwendeten Lösungsmittel leicht löslich sind, können die Gemische aus (i) und (ii) bzw. deren Lösungen unmittelbar nach ihrer Herstellung als Trimerisierungskatalysatoren eingesetzt werden. Falls die Komponenten (i) und (ii) jedoch miteinander unverträglich und im verwendeten Lösungsmittel nicht oder nur schwer löslich sind, ist es oft zweckmässig die zunächst gebildeten Suspensionen während ca. 15 bis 360 Minuten innerhalb des genannten Temperaturbereichs mechanisch zu durchmischen und einen etwa noch verbleibenden Niederschlag abzufiltrieren. Die dann vorliegende Lösung des Komplexes kann als solche eingesetzt oder im Vakuum eingeengt werden. Der zurückbleibende, lösungsmittelfreie Komplex kann schliesslich in anderen, bevorzugt aprotischen Lösungsmitteln, gelöst werden. So können beispielsweise bei Raumtemperatur ca. 0,2 molare «Lösungen von Kaliumacetat» in Toluol hergestellt werden.

Bei der Durchführung des erfindungsgemässen Verfahrens muss nicht auf eine strikte Abwesenheit von Alkohol in Lösungsmitteln für die Komplexe bzw. von Hydroxylgruppen aufweisenden Komplexbildnern (ii) geachtet werden, da die allenfalls zur Debatte stehenden äusserst geringen Mengen an gegenüber Isocyanatgruppen reaktionsfähigen Gruppen im Vergleich zu den Isocyanatgruppen des zu trimerisierenden Ausgangspolyisocyanats vernachlässigbar sind.

Die so erhaltenen erfindungswesentlichen Trimerisierungskatalysatoren können selbstverständlich für die Herstellung beliebiger Isocyanurate eingesetzt. werden. Dies bedeutet, dass sich die Katalysatoren nicht nur für das erfindungsgemässe Verfahren, sondern auch beispielsweise zur Herstellung von Isocyanuraten durch Trimerisierung von Monoisocyanaten eignen.

Beim erfindungsgemässen Verfahren werden die erfindungswesentlichen komplexen Katalysatoren als Einzelkomponenten oder als vorgebildete Komplexe, wie bereits ausgeführt, in Substanz oder als 0,005- bis 95-, vorzugsweise 0,01- bis 70-gew.-%ige Lösungen in den beispielhaft genannten Lösungsmitteln eingesetzt. Bei Verwendung von Hydroxylgruppen aufweisenden Lösungsmitteln bzw. von Komplexbildnern (ii) mit freien Hydroxylgruppen entstehen beim erfindungsgemässen Verfahren durch Reaktion mit einem Teil der Isocyanatgruppen des Ausgangspolyisocyanats Urethangruppen, was oftmals erwünscht ist, da derartige Urethangruppen einen cokatalytischen Effekt aufweisen. Die Menge an derartigen Lösungsmitteln, sofern es sich um einwertige Alkohole handelt, bzw. an Komplexbildnern sollte jedoch so begrenzt werden, dass im Reaktionsgemisch maximal 2 Mol-%, bezogen auf die Isocyanatgruppen des Ausgangspolyisocyanats, an Hydroxylgruppen vorliegen. Oft ist es auch zweckmässig, als Hydroxylgruppen aufweisende Lösungsmittel für die erfindungswesentlichen Komplexe mehrwertige Alkohole wie z.B. Ethylenglykol oder Glycerin einzusetzen, um die NCO-Funktionalität der erfindungsgemässen Verfahrensprodukte nicht durch Urethanbildung zu reduzieren. Die Menge derartiger mehrwertiger Alkohole muss selbstverständlich jedoch so begrenzt werden, dass keine in den Verfahrensprodukten schwerlöslichen Polyurethane entstehen.

Beim erfindungsgemässen Verfahren werden die erfindungswesentlichen Komplexe im allgemeinen in Mengen von 0,001 bis 0,1 Mol-%, bezogen auf Alkalimetallverbindung (i) einerseits und zu trimerisierendes Ausgangspolyisocyanat andererseits, eingesetzt. Falls beim erfindungsgemässen Verfahren aromatische Polyisocyanate ohne Mitverwendung von Lösungsmitteln trimerisiert werden, liegt die Menge der erfindungswesentlichen Katalysatoren vorzugsweise innerhalb des Bereichs von 0,001 bis 0,02 Mol-%, bei Verdünnung des aromatischen Ausgangspolyisocyanats mit einem geeigneten, aprotischen Lösungsmittel liegt die Menge des Komplexes im allgemeinen zwischen 0,01 und 0,08 Mol-%. Bei Verwendung von Ausgangspolyisocyanaten mit ausschliesslich aliphatisch gebundenen Isocyanatgruppen liegt die Menge des Katalysators im allgemeinen im Bereich von 0,01 bis 0,1 Mol-%, während bei Verwendung von Ausgangspolyisocyanaten mit cycloaliphatisch gebundenen Isocyanatgruppen vorzugsweise 0,03 bis 0,1 Mol-% des Katalysators eingesetzt werden, wobei sich alle Prozentangaben auf die Alkalimetallverbindung (i) einerseits und das Ausgangspolyisocyanat andererseits beziehen.

Beim erfindungsgemässen Verfahren können beliebige organische Polyisocyanate als Ausgangsmaterialien eingesetzt werden. Das erfindungsgemässe Verfahren eignet sich insbesondere zur teilweisen Trimerisierung der Isocyanatgruppen von Diisocyanaten mit aromatisch, aliphatisch oder cycloaliphatisch gebundenen Isocyanatgruppen des Molekulargewichtsbereichs 140–300 wie z.B.
Tetramethylendiisocyanat,
Hexamethylendiisocyanat,
1-Isocyanato-3,3,5-trimethyl-5-isocyanato-
    methyl-cyclohexan
    (Isophorondiisocyanat, abgekürzt: IPDI),
2,4- und/oder 2,6-Diisocyanatotoluol,
2,4'- und/oder 4,4'-Diisocyanatodiphenylmethan,
2,4'-Diisocyanatodicyclohexylmethan,
1-Methyl-2,4-diisocyanato-cyclohexan,
Lysinesterdiisocyanaten,
p-Xylylendiisocyanat
oder beliebige Gemische derartiger Diisocyanate. Besonders gut geeignet sind auch Gemische der beispielhaft genannten aromatischen Diisocyanate mit beispielhaft genannten aliphatischen Diisocyanaten im Gewichtsverhältnis 1:3 bis 3:1. Auch höherfunktionelle Polyisocyanate wie beispielsweise Polyisocyanatgemische, die durch Phosgenierung von Anilin/Formaldehyd-Kondensaten entstehen, können beim erfindungsgemässen Verfahren als Ausgangspolyisocyanat eingesetzt werden. Grundsätzlich, jedoch weniger bevorzugt, ist es auch möglich, NCO-Präpolymere, d.h. Umsetzungsprodukte von überschüssigen Mengen der beispielhaft genannten Diisocyanate mit mindestens difunktionellen Verbindungen mit gegenüber Isocyanatgruppen reaktionsfähigen Gruppen als Ausgangspolyisocyanate beim erfindungsgemässen Verfahren einzusetzen. Grundsätzlich können beim erfindungsgemässen Verfahren auch Gemische aus Diisocyanaten und Monoisocyanaten als Ausgangsmaterialien eingesetzt werden, um so zu interessanten Isocyanuratgruppen aufweisenden Polyisocyanaten mit einer gezielt erniedrigten NCO-Funktionalität zu gelangen. Hierbei werden die Di- und Monoisocyanate im allgemeinen in einem Molverhältnis Diisocyanat : Monoisocyanat von 1,5 : 1 bis 2,5 : 1 eingesetzt. Geeignete Monoisocyanate sind beispielsweise aliphatische Monoisocyanate mit 1 bis 18, vorzugsweise 4 bis 8 Kohlenstoffatomen wie Methylisocyanat, n-Butylisocyanat, n-Octylisocyanat oder Stearylisocyanat oder aromatische Monoisocyanate wie insbesondere Phenylisocyanat. Bevorzugte Ausgangspolyisocyanate für das erfindungsgemässe Verfahren sind 2,4- und/oder 2,6-Diisocyanatotoluol, Hexamethylendiisocyanat und IPDI.

Das erfindungsgemässe Verfahren kann in Abwesenheit oder in Gegenwart von gegenüber Isocyanatgruppen inerten Lösungsmitteln durchgeführt werden. Als Lösungsmittel für das erfindungsgemässe Verfahren eignen sich beliebige, gegenüber Isocyanatgruppen inerte Lösungsmittel bzw. Lösungsmittelgemische, deren Siedepunkt im weiten Bereich von ca. 50 °C/1013 mbar bis 250 °C/13,3 mbar liegt. Je nach Anwendungsbereich der erfindungsgemässen Verfahrensprodukte können niedrig- bis mittelsiedende Lösungsmittel oder hochsiedende angewandt werden. Bevorzugt einzusetzende Lösungsmittel sind beispielsweise Ester wie Ethylacetat, Butylacetat, Ethylenglykol-monomethylether-acetat, Ethylenglykol-monoethylether-acetat oder Ketone wie z.B. Aceton, Methylethylketon, Methylisobutylketon, Cyclohexanon oder Methoxyhexanon. Auch Phthalsäureester wie z.B. Dibutylphthalat, Butylbenzylphthalat oder Phosphorsäureester wie Trikresylphosphat oder auch Alkylsulfonsäureester des Phenols und des Kresols sind geeignet. In Verdünnungsmitteln wie z.B. Toluol, Xylol oder höheren Aromaten besteht oft nur eine begrenzte Löslichkeit, so dass höhere Zusätze solcher Verdünnungsmittel zu Trübungen und Ausfällungen in den Reaktionsprodukten führen können.

Das bei der Durchführung des erfindungsgemässen Verfahrens eingesetzte Lösungsmittel bzw. die eingesetzte Lösungsmittelmenge braucht nicht mit dem Lösungsmittel bzw. der Lösungsmittelmenge identisch zu sein, die in den erfindungsgemässen Verfahrensprodukten bei ihrer erfindungsgemässen Verwendung vorliegen. So kann man nach Beendigung des erfindungsgemässen Verfahrens das eingesetzte Lösungsmittel bzw. Lösungsmittelgemisch selbstverständlich ganz oder teilweise destillativ entfernen und ganz oder teilweise durch ein anderes Lösungsmittel ersetzen. Lösungsmittelfrei hergestellte Produkte können selbstverständlich nachträglich in den obengenannten Lösungsmitteln gelöst werden.

Das erfindungsgemässe Verfahren wird im allgemeinen im Temperaturbereich von 0 bis 200 °C, vorzugsweise zwischen 10 und 100 °C und besonders bevorzugt zwischen 25 und 80 °C durchgeführt. Bei der Durchführung des erfindungsgemässen Verfahrens in Gegenwart eines Lösungsmittels setzt man im allgemeinen das zu trimerisierende Ausgangspolyisocyanat und das betreffende Lösungsmittel in einem Gewichtsverhältnis von 1 : 4 bis 4 : 1, vorzugsweise 1 : 2 bis 2 : 1, besonders bevorzugt 0,8 : 1,2 bis 1,2 : 0,8 entsprechenden Mengen ein.

Die zu wählende Katalysatormenge richtet sich, wie oben ausgeführt, nach der Art des Ausgangspolyisocyanats und selbstverständlich auch nach der Reaktionstemperatur, bei welcher die Trimerisierung durchgeführt werden soll. Sie kann in einem einfachen orientierenden Vorversuch zuverlässig ermittelt werden. Im allgemeinen ist die Katalysatorenkonzentration gegenüber dem lösungsmittelfreien Verfahren bei Verwendung von Lösungsmitteln um den Faktor 6 bis 10 zu erhöhen.

Die Trimerisierungsreaktion kann beispielsweise nach folgenden Varianten durchgeführt werden:

1. Der Katalysator bzw. seine Lösung in einem der hierfür geeigneten Lösungsmittel wird dem zu trimerisierenden Polyisocyanat ohne Mitverwendung eines Hilfslösungsmittels für die Trimerisierungsreaktion bei Raumtemperatur zudosiert, worauf im allgemeinen die schwach exotherme Trimerisierungsreaktion spontan anspringt. Die Reaktionstemperatur wird anschliessend gegebenenfalls durch äusseres Kühlen bzw. Heizen auf der gewünschten Reaktionstemperatur gehalten, bis die Trimerisierungsreaktion durch Zugabe eines Katalysatorengiftes abgebrochen wird.

2. Das zu trimerisierende Polyisocyanat wird in einem Lösungsmittel der oben beispielhaft genannten Art vorgelegt; anschliessend wird diese Lösung mit dem Trimerisierungskatalysator bzw. seiner Lösung versetzt. Je nach Art des Ausgangspolyisocyanats und des Trimerisierungskatalysators springt bei dieser Variante die Trimerisierungsreaktion bei Raumtemperatur bzw. schwach erhöhter Temperatur innerhalb einer Inkubationszeit von ca. 0 bis 180 Minuten an. Eine derartige Inkubationszeit wird insbesondere dann beobachtet, wenn in Abwesenheit von Hydroxylgruppen aufweisenden Lösungsmitteln für den Trimerisierungskatalysator und in Abwesenheit von Hydroxylgruppen aufweisenden Komplexbildnern (ii) gearbeitet wird. Auch bei dieser Variante des erfindungsgemässen Verfahrens wird die Temperatur des Reaktionsgemischs gegebenenfalls durch äusseres Kühlen bzw. Heizen innerhalb der genannten Bereiche eingestellt. Auch hier erfolgt der Abbruch der Trimerisierungsreaktion beim Erreichen des erwünschten Trimerisierungsgrades durch Zugabe eines Katalysatorengiftes.

3. In dem als Reaktionsmedium dienenden Hilfslösungsmittel wird ein vorab hergestellter Komplex bzw. seine Lösung zunächst gelöst, worauf sich die Zugabe des zu trimerisierenden Ausgangspolyisocyanates anschliesst. Die weitere Arbeitsweise gemäss dieser Variante entspricht der oben unter 2. genannten Ausführungsform.

4. In das als Reaktionsmedium dienende Lösungsmittel werden die Katalysatorkomponenten (i) und (ii) getrennt eindosiert. Nach inniger Durchmischung wird anschliessend das zu trimerisierende Ausgangspolyisocyanat zu der entstandenen Komplexlösung gegeben. Die weitere Reaktionsführung gemäss dieser Variante entspricht ebenfalls der unter 2. genannten Ausführungsform.

Bei allen beispielhaft genannten Verfahren des erfindungsgemässen Variante wird die Trimerisierungsreaktion im allgemeinen bei Erreichen eines Trimerisierungsgrads (Trimerisierungsgrad = Prozentsatz der trimerisierten Isocyanatgruppen, bezogen auf Gesamtmenge der im Ausgangspolyisocyanat vorliegenden Isocyanatgruppen) von 10 bis 70% abgebrochen. Bei der lösungsmittelfreien Durchführung des erfindungsgemässen Verfahrens unter anschliessender Entfernung von überschüssigem Ausgangspolyisocyanat, beispielsweise im Dünnschichtverdampfer, liegt der Trimerisierungsgrad im allgemeinen zwischen 10 und 40%. Bei der Durchführung des erfindungsgemässen Verfahrens in Gegenwart von Lösungsmitteln ohne anschliessende Entfernung von nicht-umgesetztem Ausgangspolyisocyanat liegt der Trimerisierungsgrad im allgemeinen zwischen 50 und 70%.

Geeignete Katalysatorengifte sind beispielsweise beliebige Säurehalogenide, insbesondere Säurechloride wie z.B. Acetylchlorid, Benzoylchlorid, Terephthaloyldiol, Phthaloyldichlorid, Trichloracetylchlorid, Phosphortrichlorid oder Phosphortribromid oder starke, die Katalysatorenkomponente (i) neutralisierende und damit desaktivierende Säuren wie z.B. Schwefelsäure, Phosphorsäure, Chlorwasserstoff, Toluolsulfonsäure, Methansulfonsäure, Chlorsulfonsäure oder Nonafluorbutansulfonsäure. Zur Desaktivierung des Katalysators ist es ausreichend, wenn dem Reaktionsgemisch, bezogen auf die Katalysatorkomponente (i) 100 bis 110 Äquivalentprozent, d.h. im Falle von monofunktionellen Katalysatorengiften 100 bis 110 Mol-% des Katalysatorgiftes zugesetzt werden.

Um bei längerer Lagerung die Entstehung von Peroxiden aus den Komplexliganden (ii) zu verhindern, ist es empfehlenswert, den erfindungsgemässen Verfahrensprodukten geeignete Oxidationsschutzmittel zuzusetzen. Neben Phenolen bzw. substituierten Phenolen, welche im allgemeinen mit den NCO-Gruppen des Isocyanats reagieren, können auch alle anderen, aus der Polyurethanchemie bekannten Oxidationsschutzmittel des Standes der Technik eingesetzt werden. Diese Oxidationsschutzmittel werden im allgemeinen in einer Menge von 5 bis 50 Gew.-%, bezogen auf die Menge des Komplexbildners (ii) zugesetzt.

Die erfindungsgemässen Verfahrensprodukte können insbesondere im Falle der lösungsmittelfreien Durchführung des erfindungsgemässen Verfahrens in an sich bekannter Weise, beispielsweise durch Dünnschichtdestillation von überschüssigem, nicht-umgesetztem Ausgangspolyisocyanat befreit werden, so dass Isocyanuratgruppen aufweisende Polyisocyanate mit einem Gehalt an monomeren Ausgangsdiisocyanaten unter 3 Gew.-%, vorzugsweise unter 0,7 Gew.-%, erhältlich sind.

Die erfindungsgemässen Verfahrensprodukte können selbstverständlich in an sich bekannter Weise mit geeigneten Blockierungsmitteln für Isocyanatgruppen wie z.B. Phenol, ε-Caprolactam, Malonsäurediethylester oder Acetessigsäureethylester blockiert werden.

Die erfindungsgemässen Verfahrensprodukte bzw. ihre durch die genannte Blockierungsreaktion erhaltenen Derivate stellen wertvolle Ausgangsmaterialien zur Herstellung von Polyurethankunststoffen nach dem Isocyanat-Polyadditionsverfahren dar. Sie eignen sich insbesondere als Isocyanatkomponente in Zweikomponentenpolyurethanlacken.

Das erfindungsgemässe Verfahren zeichnet sich gegenüber den bekannten Verfahren zur Herstellung von Isocyanuratgruppen aufweisenden Polyisocyanaten durch folgende Vorteile aus:

1. Es werden nur extrem geringe Mengen Alkalimetallverbindungen als Katalysatoren eingesetzt.

2. Die Reaktion wird durch geringe Mengen an Katalysatorgiften (Säurechloriden, Imidoylchloriden bzw. Protonensäuren) zum Stillstand gebracht. Ausfällungen von Salzen werden hierbei nicht beobachtet.

3. Die schwach exotherme Reaktion ist sicherheitstechnisch und verfahrenstechnisch kontinuierlich und diskontinuierlich leicht zu beherrschen.

4. Die Reaktionszeiten sind kurz.

5. Die Verfahrensprodukte sind farblos bis leicht gelblich, durchsichtig und lagerstabil. Flüssige Produkte besitzen im Vergleich zu denen des Standes der Technik vergleichbare oder niedrigere Viskositäten, feste Produkte (z.B. aus IPDI), vergleichbare oder niedrigere Schmelzpunkte.

Die folgenden Beispiele erläutern die Erfindung. Alle Prozentangaben betreffen, soweit nicht anders lautend ausgeführt, Gewichtsprozente.

Beispiel 1

Eine Lösung von 0,0025 g (0,0025 Mol-%) Kaliumacetat und 0,015 g Polyethylenglykoldimethylether (Molgewicht 410) in 0,0325 g Ethanol wird bei 23 °C mit 174 g Toluylendiisocyanat (65% 2,4- und 35% 2,6-Isomeres) vermischt. In dem gegen Wärmeverluste isolierten Reaktionsgemisch steigt die Temperatur innerhalb 105 Minuten auf 50 °C und wird anschliessend durch Aussenkühlung bei 50 °C gehalten. Nach insgesamt 2 Stunden Reaktionsdauer wird die Reaktion durch Zusatz von 0,004 g Acetylchlorid abgestoppt. Das Produkt besitzt einen NCO-Gehalt von 41,6%. Durch Abdestillieren des monomeren Toluylendiisocyanates am Dünnschichtverdampfer im Ölpumpenvakuum erhält man einen hellgelben Feststoff, der in Butylacetat löslich ist.

Beispiel 2

Bei 25 °C werden 174 g Toluylendiisocyanat (65% 2,4- und 35% 2,6-Isomeres) unter Rühren mit einer Lösung von 0,02 g Natriumacetat (0,025 Mol-%) und 0,15 g Polyethylenglykol (mittleres Molgewicht 380) in 0,33 g Ethanol versetzt. Die Temperatur steigt innerhalb 90 Minuten auf 40 °C und wird durch Kühlung, später durch Heizen bei 40 °C gehalten. Nach insgesamt 105 Minuten Reaktionsdauer wird die Trimerisation bei 41,6% NCO-Gehalt durch Zusatz von 0,08 g Acetylchlorid und 30 Minuten Nachrühren abgebrochen.

Beispiel 3

Zu 174 g Butylacetat werden eine Lösung von 0,02 g Kaliumacetat (0,02 Mol-%) in 0,18 g Polyethylenglykol (mittleres Molgewicht 380) und 174 g destilliertes 2,4-Toluylendiisocyanat bei 25 °C gegeben. Die exotherme Reaktion springt sofort an, die Innentemperatur steigt in 5 Minuten auf 40 °C. Durch Kühlen bzw. Heizen wird die Temperatur bei 40 °C gehalten. Nach 2 Stunden Reaktionsdauer wird die Reaktion bei einem NCO-Gehalt von 7,8% durch Zusatz von 0,04 g Acetylchlorid abgebrochen. Es wird noch 60 Minuten bei 40 °C nachgerührt. Die farblose, niedrigviskose Lösung enthält noch 0,6% monomeres Toluylendiisocyanat.

Beispiel 4

Beispiel 3 wird wiederholt, jedoch die Reaktionstemperatur durch Kühlen bzw. schwaches Erwärmen bei 30 °C gehalten. Man erhält nach 3,5 Stunden Reaktionsdauer, Abstoppen der Reaktion durch Zusatz von 0,04 g Acetylchlorid und 60 Minuten Nachrühren eine farblose niederviskose Lösung mit einem NCO-Gehalt von 8,2%.

Beispiel 5

Bei 25 °C werden 174 g Butylacetat, eine Lösung von 0,046 g Natriumphenolat (0,04 Mol-%) in 0,414 g Polyethylenglykol (mittleres Molgewicht 380) und 174 g 2,4-Toluylendiisocyanat miteinander verrührt. Nach einer Inkubationszeit von 2,5 Stunden springt die Zyklotrimerisationsreaktion im gegen Wärmeverlust isolierten Reaktionsgefäss an. Die Temperatur im Reaktionsgemisch liegt nach insgesamt 3,5 Stunden bei 28 °C, nach 4 Stunden bei 34 °C und nach 4,25 Stunden bei 43 °C. Durch Kühlung wird die Temperatur bei 40 °C gehalten. Nach einer Gesamtreaktionszeit von 5 Stunden wird die Reaktion durch Zusatz von 0,034 g Acetylchlorid abgestoppt. Das flüssige Produkt enthält noch 8,2 Gew.-% NCO.

Beispiele 6 bis 13

Es wird verfahren wie in Beispiel 3. Experimentelle Details s. Tabelle 1.

Beispiel 14

Eine Lösung von 50 mg Kaliumacetat (0,017 Mol) und 300 mg eines Polyethers auf Basis Butanol/Ethyloxid (Molgewicht 515) in 650 mg Methanol wird bei 24 °C zu 504 g reinem Hexamethylendiisocyanat gegeben. Die Reaktion springt ohne Induktionsperiode sofort an. Im gegen Wärmeverluste isolierten Reaktionsgemisch wird innerhalb 3 Stunden eine Temperatur von 58 °C erreicht. Nach insgesamt 4,5 Stunden Dauer wird die Reaktion durch Zusatz von 0,08 g Acetylchlorid (0,001 Mol) und 30 Minuten Nachrühren bei 55 °C abge-

Tabelle 1
Trimerisation von 174 g 2,4-Toluylendiisocyanat in 174 g Butylacetat mit Kaliumacetat

| Beispiel Nr. | Kalium- acetat (g) | Komplex- ligand Art | Menge (g) | Kosolvenz f. Art | Katal. u. Lig. Menge (g) | Reakt.- zeit (h) | Reakt.- temp. (°C) | Produkt: NCO-Gehalt (Gew.-%) |
|---|---|---|---|---|---|---|---|---|
| 6 | 0,018 | A[1] | 0,071 | – | – | 12 | max. 50 | 8,2 |
| 7 | 0,03 | B[2] | 0,18 | EtOH[8] | 0,39 | 4 | 60 | 8,1 |
| 8 | 0,02 | C[3] | 0,16 | EtOH[8] | 0,22 | 7 | 50 | 8,2 |
| 9 | 0,03 | D[4] | 0,31 | EtOH[8] | 0,26 | 7 | 50 | 8,2 |
| 10 | 0,02 | E[5] | 0,12 | EtOH[8] | 0,26 | 1 | 50 | 8,2 |
| 11 | 0,01 | E[5] | 0,06 | EtOH[8] | 0,13 | 10 | 40 | 8,1 |
| 12 | 0,02 | F[6] | 0,12 | MeOH[9] | 0,26 | 1,25 | 50 | 8,1 |
| 13 | 0,02 | G[7] | 0,18 | – | – | $2^1/_3$ | 50 | 8,2 |

[1] Oktaethylenglykoldimethylether;
[2] Dimethylether von Polyethylenglykol ($\overline{MG}$ 380);
[3] Dimethylether von Polyethylenglykol ($\overline{MG}$ 606);
[4] Trimethylether von Polyether (aus Trimethylolpropan und Ethylenoxid, $\overline{MG}$ 673);
[5] Bis-urethan aus 2 Mol Methylisocyanat und 1 Mol Polyethylenglykol ($\overline{MG}$ 380);
[6] Polyetheralkohol aus Butanol und Ethylenoxid ($\overline{MG}$ 515);
[7] Polyethylenglykol mit mittlerem Molekulargewicht $\overline{MG}$ 380;
[8] Ethanol;
[9] Methanol.

stoppt. Das rohe isocyanurathaltige Produkt enthält noch 41,1% NCO. Nach Destillation am Dünnschichtverdampfer unter Stickstoff bei 0,5 Torr und 160 °C werden 170 g farbloses, niedrigviskoses Produkt mit 22,2% Isocyanatgehalt und 0,7% Gehalt an monomerem Hexamethylendiisocyanat erhalten.

Beispiel 15

Zu 4032 g destilliertem Hexamethylendiisocyanat (24 Mol) wird bei 25 °C eine Lösung von 0,96 g Kaliumacetat (9,8 × $10^{-3}$ Mol = 0,04 Mol-%) in 8,64 g Polyethylenglykol (mittleres Molgewicht 380) zugesetzt. Innerhalb 40 Minuten steigt die Temperatur des Reaktionsgemisches auf 51 °C, nach weiteren 60 Minuten auf 60 °C. Die Temperatur wird nun bei 60 °C gehalten. Nach einer Gesamtreaktionszeit von 4,5 Stunden wird die Trimerisierung durch Zusatz von 1,0 g Acetylchlorid und 60 Minuten Nachrühren bei Raumtemperatur abgestoppt.

Das Rohprodukt besitzt einen NCO-Gehalt von 40,8%. Nach Entfernung des monomeren Hexamethylendiisocyanates durch Hochvakuumdestillation im Dünnschichtverdampfer werden 1350 g eines niederviskosen Produktes mit η (25 °C) = 2500 mPas erhalten. Der NCO-Gehalt beträgt 22,6%, der Gehalt an monomerem Hexamethylendiisocyanat noch 0,11%.

Beispiel 16

Zu 4032 g reinem Hexamethylendiisocyanat (24 Mol) wird bei 25 °C eine Lösung von 0,96 g Kaliumacetat in 8,64 g Polyethylenglykol (Molgew. 380) unter Rühren zugesetzt. Die schwach exotherme Reaktion springt sofort an und innerhalb von 20 Minuten ist die Temperatur im Reaktionsgemisch auf 63 °C gestiegen. Durch gelegentliches Kühlen wird die Innentemperatur bei 60 °C gehalten und nach einer Gesamtreaktionszeit von 75 Minuten wird die Reaktion durch Zusatz von 1,0 g Acetylchlorid bei einem NCO-Gehalt von 40,5% NCO abgestoppt. Nach Hochvakuumdestillation im Dünnschichtverdampfer weist das hellgelbe Produkt (1400 g) bei einem NCO-Gehalt von 22,9% und einem Gehalt an monomerem Hexamethylendiisocyanat von 0,2% eine Viskosität von 2800 mPas/25 °C auf.

Beispiele 17 bis 20

Es wird verfahren wie in Beispiel 14. Experimentelle Angaben s. Tabelle 2.

Beispiel 21

Bei 60 °C werden zu 222 g Isophorondiisocyanat (IPDI, 1 Mol) unter Rühren eine Lösung von 0,3 g Kaliumacetat (0,3 Mol-%) in 2,7 g Polyethylenglykol (Molgewicht 380) zugesetzt. Nach anfänglicher Trübung wird das Reaktionsgemisch im Verlauf der Reaktion wieder klar. Es wird 18 Stunden bei 60 °C weitergerührt. Danach wird die Zyklotrimerisation bei einem NCO-Gehalt von 30,2% durch Zusatz von 0,26 g Acetylchlorid und eine Stunde Rühren bei 60 °C abgebrochen.

Nach Abdestillieren des monomeren IPDI am Dünnschichtverdampfer bei 160 °C und 0,1 Torr erhält man einen hellgelben Feststoff mit 0,5% monomerem IPDI und einem Schmelzbereich von 121 bis 127 °C.

Tabelle 2
Zyklotrimerisation von 504 g Hexamethylendiisocyanat (3 Mol) in Substanz mit Kaliumacetat

| Beispiel Nr. | Kalium-acetat (g) | Komplex-ligand Art | Menge (g) | Kosolvenz f. Art | Katal. u. Lig. Menge (g) | Reakt.-zeit (h) | Reakt.-temp. (°C) | Roh-Produkt: NCO-Gehalt (Gew.-%) |
|---|---|---|---|---|---|---|---|---|
| 17 | 0,25[1] | B | 1,97 | $CH_2Cl_2$[2] | 350[2] (250 ml) | 12 | 40–50 | 40,7 |
| 18 | 0,05 | B | 0,3 | EtOH | 0,65 | 5 | 50 | 41,1 |
| 19 | 0,05 | H[3] | 0,3 | EtOH | 0,65 | 8 | 50–55 | |
| 20 | 0,3 | F | 1,8 | MeOH | 3,9 | 1,5 | 60 | 41,1 |

[1] Kaliumacetat, B mit 250 ml $CH_2Cl_2$ 2 Stunden heftig gerührt, abfiltriert, Lösung eingesetzt;
[2] zu Reaktionsbeginn im Vakuum abdestilliert;
[3] Bis-urethan aus 2 Mol Phenylisocyanat und 1 Mol G.

**Patentansprüche**

1. Verfahren zur Herstellung von Isocyanuratgruppen aufweisenden Polyisocyanaten durch Trimerisierung eines Teils der Isocyanatgruppen von organischen Polyisocyanaten oder Gemischen aus Di- und Monoisocyanaten in Gegenwart von basischen Alkalimetallverbindungen als Katalysatoren und gegebenenfalls in Gegenwart von Hilfslösungsmitteln und Abbruch der Trimerisierungsreaktion durch Zugabe eines Katalysatorengiftes, dadurch gekennzeichnet, dass man als Trimerisierungskatalysatoren Komplexe aus
i) basischen Alkalimetallverbindungen und
ii) acyclischen organischen Verbindungen, die
   a) insgesamt mindestens 7 Ethylenoxid- und gegebenenfalls Propylenoxideinheiten in Form einer oder mehrerer Polyetherketten mit jeweils mindestens 3 Alkylenoxideinheiten, wobei mindestens 80% aller vorliegender Alkylenoxideinheiten Ethylenoxideinheiten darstellen,
   b) einen Gehalt an innerhalb mindestens einer Polyetherkette angeordneten Alkylenoxideinheiten der genannten Art von insgesamt mindestens 40 Gew.-% und
   c) ein Molekulargewicht von 326–1000 aufweisen,
in Mengen von 0,001 bis 0,1 Mol.-%, bezogen auf Alkalimetallverbindung (i) einerseits und zu trimerisierendes Ausgangspolyisocyanat andererseits, verwendet, mit der Massgabe, dass im Falle der Verwendung von Komplexbildnern (ii) mit freien Hydroxylgruppen bzw. an einwertigen Alkoholen als Lösungsmittel deren Menge so begrenzt wird, dass im Reaktionsgemisch maximal 2 Mol-%, bezogen auf die Isocyanatgruppen des Ausgangspolyisocyanats, an Hydroxylgruppen vorliegen.

2. Verfahren gemäss Anspruch 1, dadurch gekennzeichnet, dass man als Trimerisierungskatalysatoren solche Komplexe verwendet, deren Komponente (ii) aus mindestens einem ein- oder mehrwertigen Polyetheralkohol besteht, dessen endständige(n) Hydroxylgruppe(n) gegebenenfalls in blockierter Form vorliegen.

3. Verfahren gemäss Ansprüchen 1 und 2, dadurch gekennzeichnet, dass man als Trimerisierungskatalysatoren solche Komplexe verwendet, deren Komponente (i) aus einem Natrium- oder Kaliumcarboxylat mit 1 bis 18 Kohlenstoffatomen besteht.

4. Verfahren gemäss Ansprüchen 1 bis 3, dadurch gekennzeichnet, dass man als Trimerisierungskatalysator solche Komplexe verwendet, die durch Umsetzung der Komponente (i) mit mindestens einer äquimolaren Menge der Komponente (ii) bei 10 bis 60 °C erhalten worden sind.

5. Verfahren gemäss Ansprüchen 1 bis 4, dadurch gekennzeichnet, dass man den als Trimerisierungskatalysator eingesetzten Komplex dem zu trimerisierenden Polyisocyanat in Form einer Lösung in einem gegenüber Isocyanatgruppen inerten Lösungsmittel oder in Form einer Lösung in einer gegenüber Isocyanatgruppen reaktionsfähige Gruppen aufweisenden Flüssigkeit zudosiert.

6. Verfahren gemäss Ansprüchen 1 bis 5, dadurch gekennzeichnet, dass man die Umsetzung im Temperaturbereich von 10 bis 100 °C unter Verwendung von 0,001 bis 0,5 Mol-%, bezogen auf Komponente (i) einerseits und die Menge des teilweise zu trimerisierenden Polyisocyanats andererseits, durchführt.

7. Verwendung der gemäss Ansprüchen 1 bis 6 erhältlichen Isocyanuratgruppen aufweisenden Polyisocyanate, gegebenenfalls in von überschüssigen Ausgangspolyisocyanaten befreiter Form und/oder gegebenenfalls in mit Blockierungsmitteln für Isocyanatgruppen blockierter Form als Isocyanatkomponente zur Herstellung von Polyurethanen nach dem Isocyanat-Polyadditionsverfahren.

**Claims**

1. Process for the production of polyisocyanates containing isocyanurate groups by trimerising a proportion of the isocyanate groups of organic polyisocyanates or mixtures of di- and monoisocyanates in the presence of basic alkali metal compounds as catalysts and optionally in the presence of auxiliary solvents and stopping the trimerisation reaction by adding a catalyst poison, characterised in that complexes of
i) basic alkali metal compounds and
ii) acyclic organic compounds which

a) have altogether at least 7 ethylene oxide and optionally propylene oxide units in the form of one or more polyether chains each with at least 3 alkylene oxide units, at least 80% of all of the alkylene oxide units present being ethylene oxide units,

b) have a content of altogether at least 40% by weight of alkylene oxide units of the type mentioned arranged within at least one polyether chain and

c) have a molecular weight of 326–1.000, are used as the trimerisation catalysts, in amounts of 0.001 to 0.1 mol %, based on the alkali metal compound (i) on the one hand and the starting polyisocyanate to be trimerised, on the other hand, with the proviso that, in the case of the use of complexing agents (ii) with free hydroxyl groups or of monofunctional alcohols as solvents, the amount thereof is limited in such a manner that at most 2 mol %, based on the isocyanate groups of the starting polyisocyanate, of hydroxyl groups are present in the reaction mixture.

2. Process according to Claim 1, characterised in that those complexes in which component (ii) consists of at least one mono- or polyfunctional polyether alcohol, the terminal hydroxyl group(s) of which is/are optionally present in blocked form, are used as the trimerisation catalysts.

3. Process according to Claims 1 and 2, characterised in that those complexes in which component (i) consists of a sodium or potassium carboxylate with 1 to 18 carbon atoms are used as the trimerisation catalysts.

4. Process according to Claims 1 to 3, characterised in that those complexes which have been obtained by reacting component (i) with an at least equimolar amount of component (ii) at 10 to 60 °C are used as the trimerisation catalyst.

5. Process according to Claims 1 to 4, characterised in that the complex used as the trimerisation catalyst is metered into the polyisocyanate to be trimerised in the form of a solution in a solvent which is inert towards isocyanate groups or in the form of a solution in a liquid containing groups which are reactive towards isocyanate groups.

6. Process according to Claims 1 to 5, characterised in that the reaction is carried out in the temperature range of 10 to 100 °C using 0.001 to 0.5 mol%, based on component (i) on the one hand and the amount of the polyisocyanate to be partially trimerised on the other hand.

7. Use of the polyisocyanates containing isocyanurate groups obtainable according to Claims 1 to 6, optionally in a form which is free from excess starting polyisocyanates and/or optionally in a form which is blocked with blocking agents for isocyanate groups, as the isocyanate component for the production of polyurethanes by the isocyanate polyaddition process.

**Revendications**

1. Procédé de préparation de polyisocyanates contenant des groupes isocyanurates par trimérisation d'une partie des groupes isocyanates de polyisocyanates organiques ou de mélanges de di- et de mono-isocyanates en présence de composés de métaux alcalins basiques qui servent de catalyseurs et le cas échéant, en présence de solvants auxiliaires, et interruption de la réaction de trimérisation par addition d'un poison de catalyseur, caractérisé en ce que l'on utilise en tant que catalyseurs de trimérisation des complexes de:

i) des composés de métaux alcalins basiques et

ii) des composés organiques acycliques qui

a) contiennent au total au moins 7 motifs d'oxyde d'éthylène et le cas échéant d'oxyde de propylène sous forme d'une ou plusieurs chaînes de polyéther contenant chacune au moins 3 motifs d'oxydes d'alkylène, 80% au moins de tous les motifs d'oxydes d'alkylène présents étant des motifs d'oxyde d'éthylène,

b) contiennent au total au moins 40% en poids des motifs d'oxydes d'alkylène du type mentionné disposés à l'intérieur d'au moins une chaîne de polyéther, et

c) ont un poids moléculaire de 326 à 1000, en quantités de 0,001 à 0,1 mol %, par rapport au composé de métal alcalin (i) d'une part et au polyisocyanate de départ à trimériser d'autre part, étant spécifié que dans le cas où on utilise des complexants (ii) portant des groupes hydroxy libres ou des alcools monovalents en tant que solvants, leur quantité doit être limitée au point que le mélange de réaction contienne au maximum 2 mol % de groupes hydroxy par rapport aux groupes isocyanates du polyisocyanate de départ.

2. Procédé selon la revendication 1, caractérisé en ce que l'on utilise en tant que catalyseurs de trimérisation des complexes dont le composant (ii) consiste en au moins un polyéther-alcool mono- ou poly-valent dont le ou les groupes hydroxy terminaux sont éventuellement à l'état bloqué.

3. Procécé selon les revendications 1 et 2, caractérisé en ce que l'on utilise en tant que catalyseurs de trimérisation des complexes dont le composant (i) consiste en un carboxylate de sodium ou de potassium en $C_1$–$C_{18}$.

4. Procédé selon les revendications 1 à 3, caractérisé en ce que l'on utilise en tant que catalyseurs de trimérisation des complexes qui ont été obtenus par réaction du composant (i) avec la quantité au moins équimoléculaire du composant (ii) à une température de 10 à 60 °C.

5. Procédé selon les revendications 1 à 4, caractérisé en ce que l'on ajoute le complexe mis en œuvre en tant que catalyseur de trimérisation au polyisocyanate à trimériser à l'état de solution dans un solvant inerte à l'égard des groupes isocyanates ou à l'état de solution dans un liquide portant des groupes réactifs à l'égard des groupes isocyanates.

6. Procédé selon les revendications 1 à 5, caractérisé en ce que la réaction est effectuée dans l'intervalle de température de 10 à 100 °C avec utilisation de 0,001 à 0,5 mol %; par rapport au composant (i) d'une part et à la quantité du polyisocyanate à trimériser en partie d'autre part.

7. Utilisation des polyisocyanates portant des groupes isocyanurates obtenus par un procédé selon les revendications 1 à 6, éventuellement débarrassés de l'excès de polyisocyanate de départ et/ou éventuellement à l'état bloqué par des agents bloquants des groupes isocyanates, en tant que composant isocyanate dans la préparation de polyuréthannes par le procédé de polyaddition des isocyanates.